(19) Europäisches Patentamt
European Patent Office
Office européen des brevets

(11) EP 1 437 143 A1

(12) **EUROPEAN PATENT APPLICATION**
published in accordance with Art. 158(3) EPC

(43) Date of publication:
**14.07.2004 Bulletin 2004/29**

(21) Application number: **02775260.9**

(22) Date of filing: **27.09.2002**

(51) Int Cl.[7]: **A61K 47/48**, A61K 47/34, A61K 45/00, A61K 9/08, A61P 27/02, A61P 27/06

(86) International application number:
**PCT/JP2002/010123**

(87) International publication number:
**WO 2003/028765 (10.04.2003 Gazette 2003/15)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **28.09.2001 JP 2001299193**

(71) Applicant: **SANTEN PHARMACEUTICAL CO., LTD.**
**Higashiyodogawa-ku, Osaka-shi,**
**Osaka 533-8651 (JP)**

(72) Inventors:
- **TASAKA, Humitaka,**
**SANTEN PHARMACEUTICAL CO., LTD.**
**Ikoma-shi, Nara 630-0101 (JP)**
- **NAKAGAWA, Masaki,**
**SANTEN PHARMACEUTICAL CO., LTD.**
**Ikoma-shi, Nara 630-0101 (JP)**
- **HORIBE, Yoshihide,**
**SANTEN PHARMACEUTICAL CO., LTD.**
**Ikoma-shi, Nara 630-0101 (JP)**
- **KUWANO, Mitsuaki,**
**SANTEN PHARMACEUTICAL CO., LTD.**
**Ikoma-shi, Nara 630-0101 (JP)**

(74) Representative:
**TER MEER STEINMEISTER & PARTNER GbR**
**Patentanwälte,**
**Mauerkircherstrasse 45**
**81679 München (DE)**

# (54) INJECTIONS FOR EYE TISSUE CONTAINING DRUG BOUND TO POLYETHYLENE GLYCOL

(57) The present invention relates to injections for ocular tissues containing a conjugate covalently bonded a drug to PEG. When the drug-PEG conjugate is injected into the ocular tissues, the drug can be retained in the ocular tissues such as an iris, a ciliary body, a vitreous body, a retina and an optic nerve for a long period. Accordingly, the injections for ocular tissues of the present invention make it possible to treat or prevent diseases in various ocular tissues over a long period by a single administration. PEG can be any of straight-chain, stellate and branched. In the case of straight-chain PEG, since the drugs are generally bonded using hydroxyl groups at its both ends, a binding ratio of the drug to PEG is 1:1 or 2:1. Since stellate and branched PEGs have plural hydroxyl groups, plural covalent bond of drugs to PEG can be formed.

EP 1 437 143 A1

## Description

Technical Field

The present invention relates to injections for eye tissues containing a drug-polyethylene glycol conjugate which are retained in the ocular tissues for a long period.

Background Art

Diseases in eye tissues such as an iris, a ciliary body, a retina, an optic nerve or a vitreous body are often intractable, and a development of an effective treatment method is eagerly desired. Though ocular diseases are most generally treated by instillation of drugs, delivery of the drugs is difficult in some ocular tissues, particularly the drugs are hardly delivered to the intraocular tissues such as a vitreous body and a retina, rendering the treatment of the intraocular diseases all the more difficult. Since it is difficult to obtain sustainment of the drugs by instillation, frequent administration is required.

In view of this, a method of administering a drug directly to ocular tissues was attempted. For example, a technique for administering a liposome or a microsphere containing a drug to intraocular tissues such as a vitreous body was reported (Published Japanese Translation of PCT No. 508369/1994, Japanese Laid-open Patent Publication No. 221322/1992 and the like).

However, it is not easy to control the release of drugs by using the liposome. The liposome and the microsphere have large particle diameters. Accordingly, when they are administered to the intraocular tissues such as the vitreous body, transparency in the vitreous body sometimes cannot be maintained.

On the other hand, it is known that conjugates covalently bonded drugs to polyethylene glycol (PEG) improve retentivity of the drugs in bodies and the drug-PEG conjugates are useful as drug delivery systems. Various specific drug-PEG conjugates have been synthesized, and insulin-PEG conjugates (US 4,179,337), taxol-PEG conjugates (WO 93/24476), interferon-PEG conjugates (WO 99/48535), asparaginase-PEG conjugates (WO 99/39732) and urate oxidase-PEG conjugates (WO 00/7629) are known. However, none of them is aimed at use in an ophthalmic field.

With regard to application of the drug-PEG conjugates in the ophthalmic field, it is reported that hydrocortisone-PEG conjugates improve scleral penetration compared with hydrocortisone itself (Int. J. Pharm., 182 (1), 79-92, 1999). It is also reported that intravenous administration of a superoxide dismutase (SOD)-PEG conjugate to rats improved retentivity of SOD and inhibited ischemia-induced retinal edema (Invest. Ophthalmol. Vis. Sci., 32, 1471-1478, 1991).

However, there has been no report concerning techniques for injecting the drug-PEG conjugates into ocular tissues. Accordingly, no effect owing to the injection into ocular tissues is of course known at all.

Though it is known that the above-mentioned drug-PEG conjugates are useful as the drug delivery systems, a conventional method of administering them intravenously or orally or instillation them causes many problems in treating diseases in various ocular tissues, particularly intraocular tissues. For example, since the drug-PEG conjugate administered intravenously spreads systemically with blood stream, an amount of the drug which reaches the ocular tissues is far smaller than a dosage. Accordingly, a large amount of the drug must be administered in order to make an effective amount of the drug reach to the ocular tissues. However, in this case, systemic side-effects could become a serious issue. Further, since the drug-PEG conjugates administered intravenously has the problem that they are subject to metabolism in blood, frequent administration is required in treatment. In the case of oral administration, since a metabolism process in a liver is added, the amount of the drug which reaches the ocular tissues further decreases. Even in the case of instillation, which is local administration, the amount of the drug which reaches the intraocular tissues such as the retina through a cornea is about 1/10,000 as large as the dosage because of a barrier mechanism of corneal epithelium.

Disclosure of the Invention

The present inventors found that when a drug-PEG conjugate is directly administered to ocular tissues, the drug can directly reach the ocular tissues such as an iris, a ciliary body, a vitreous body, a retina and an optic nerve, and the drug can be retained in the ocular tissues for a long period, accordingly the conjugate is useful for treatment of diseases in various ocular tissues. When the drug-PEG conjugate is directly administered to the ocular tissues, the conjugate does not enter systemic circulation. Accordingly, almost all of a dosage is utilized for treatment of the diseases in the ocular tissues, and systemic side-effects are reduced. The drug is administered in the form of conjugate with PEG. However, the bond of the drug with PEG is gradually broken after injecting it into the ocular tissues so that release of the drug may be controlled, thereby making it possible to exhibit therapeutic effects on the diseases over a long period. The drug-PEG conjugate is excellent in retentivity in the ocular tissues. Even if the bond of the drug with PEG

is not broken in the ocular tissues and kept, the conjugate can exhibit therapeutic effects on the diseases in the various ocular tissues. Accordingly, the injection into the ocular tissues on the present invention makes it possible by a single administration to treat the diseases in the various ocular tissues, in particular, which have been difficult to treat so far.

The present invention relates to injections for the ocular tissues containing a conjugate covalently bonded a drug to PEG. When the drug-PEG conjugate is injected into the ocular tissues, the conjugate is retained in the ocular tissues such as an iris, a ciliary body, a retina, an optic nerve and a vitreous body for a long period, thereby making it possible to sustain drug efficacy over a long period by a single administration.

PEG can be any one of straight-chain, stellate and branched in the present invention. In straight-chain PEG, since the drugs are generally bonded to PEG using hydroxyl groups at its both ends, a bonding ratio of the drug to PEG is 1:1 or 2:1. Since stellate and branched PEGs have plural hydroxyl groups, plural covalent bond of drug to PEG can be formed. Molecular weight of PEG is not particularly limited and can appropriately be selected considering the kind and properties of the drug forming the covalent bond, a period for retaining the drug, and the like. The molecular weight is usually 300 to 200,000, more preferably 1,000 to 50,000.

Drugs having a functional group such as carboxyl can be directly covalently bonded to PEG using the hydroxyl groups at the ends of PEG. In accordance with the kind of drug, PEG can also be converted into a derivative having functional groups such as amino, thiol and carboxyl in advance so that covalent bonds to the drug can be easily formed. Namely, in accordance with the kind of drug, PEG is converted into the derivative having the functional groups such as amino, thiol and carboxyl using the hydroxyl groups of PEG, and then the drug can be covalently bonded to the obtained derivative.

Both hydroxyl groups at the ends of PEG can be used in order to form the covalent bonds. When only one hydroxyl group is used for bonding, the other hydroxyl group which does not participate in the bond can be protected with a protecting group such as alkyl or acyl. When PEG is converted into the derivative having the functional groups, either both functional groups or only one functional group can be used in order to form the covalent bonds.

These bonds are schematically shown below. (In each formula, X is the drug, and R is hydrogen, a protecting group such as alkyl or acyl, carboxyalkyl, aminoalkyl or the like.)

- A state in which no drug is bonded to PEG (PEG only):

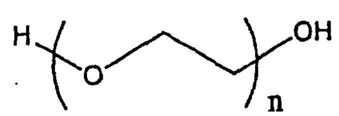

- One bond of drug to the hydroxyl group of PEG:

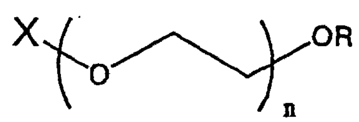

- Two bonds of drug to the hydroxyl groups of PEG:

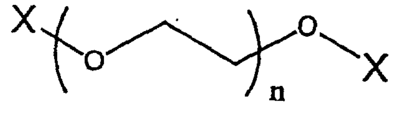

- One bond of drug to an amino derivative of PEG

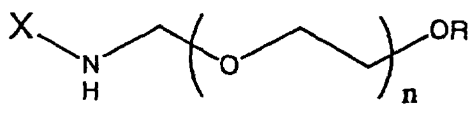

- One bond of drug to a carboxyl derivative of PEG

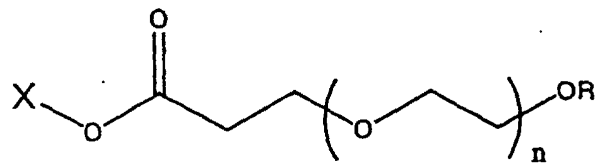

- One bond of drug to a thiol derivative of PEG

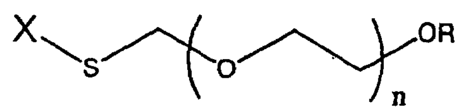

Widely-used processes can be used in order to form the above-mentioned covalent bonds, and is exemplified by a process for esterifying the carboxylic acid derivative of PEG with a hydroxyl group of the drug.

Chemical structure of the drug to be covalently bonded to PEG is not limited. Drugs can be used which have a functional group which can be bonded to PEG. Specific examples of drugs are ones having hydroxyl, carboxyl, carbonyl, amino or alkenyl. The drugs can be converted into derivatives having a functional group such as amino, thiol, carboxyl or isothiocyanate in advance so that the covalent bond to PEG can be easily formed. The kind of the drugs is not limited so far as the drugs are ones having therapeutic effects or preventive effects on ophthalmopathy. Examples of the drugs are anti-inflammatories, immunosuppressors, antivirals, antimicrobials, antimycotics, antitumors, nerve-protecting drugs, bloodflow-improving drugs, antiglaucomatous drugs, analgesics, anesthetics, angiogenesis inhibitors and diagnostic agents. In particular, examples of drugs for intraocular diseases of an iris, a ciliary body, a retina, an optic nerve, a vitreous body are drugs which are effective for intraocular inflammation due to various causes, viral or bacterial infections, proliferative viteoretinopathy accompanied by proliferation of retinal cells, retinal neovasculaturigation, retinal hemorrhage due to various causes, retinal detachment or retinoblastoma. For example, anti-inflammatories such as betamethasone phosphate are used for treating inflammation accompanying an intraocular surgical operation. Immunosuppressors such as cyclosporin are used for treating autoimmune uveitis. Antivirals such as ganciclovir are used for treating viral infections. Antimicrobials such as ofloxacin are used for treating postoperative infections. Antitumors such as doxorubicin hydrochloride and carmustine are used for treating proliferative viteoretinopathy. Ophthalmic diagnostic agents are used for various inspections.

Examples of methods of injecting the drug-PEG conjugate into the ocular tissues are a subretinal injection, an intravitreous injection, an intrascleral injection, an anterior chamber injection and a sub-Tenon's injection.

Effects of the present invention will be described later in detail in intraocular pharmacokinetics tests and pharmacological tests. In brief, in the intraocular pharmacokinetics tests, a fluorescein-PEG conjugate was studied for retentivity of the drug-PEG conjugate in intraocular tissues (a vitreous body and a retina) after injection into the vitreous body. As a result, it was found that the drug is retained in the vitreous body and the retina over a long period by virtue of the injection for ocular tissues of the present invention. Further, in the pharmacological tests, a betamethasone-PEG conjugate was injected into a vitreous body or under a conjunctiva once, and effects on krypton laser-induced choroidal neovascularization were studied. As a result, it was found that choroidal neovascularization is inhibited by virtue of the injection for ocular tissues of the present invention, and the drug-PEG conjugate is useful for treatment of ophthalmopathy.

These test results show that various intraocular diseases can be treated effectively with a fewer times of administration by selecting appropriately the drug bonded to PEG. The injection for ocular tissues of the present invention makes the drug retained efficiently at the intraocular tissues such as an iris, a ciliary body, a retina, an optic nerve and a vitreous body. Accordingly, it is possible to reduce an amount of the drug, and an effect of reducing side-effects can be expected.

Preparation forms of the drug-PEG conjugate in the injection for ocular tissues of the present invention are preferably liquid preparations. The liquid preparations can be prepared, for example, by dissolving the drug-PEG conjugate in BSS (Balanced Salt Solution), a glycerin solution, a hyaluronic acid solution and the like. A stabilizer, an isotonic agent, a buffer, a pH adjustor, a soothing agent, a preservative or the like can optionally be added in an adequate amount to the liquid preparations.

An example of stabilizers is disodium edetate or the like. Examples of isotonic agents are glycerin, propylene glycol, polyethylene glycol, sodium chloride, potassium chloride, sorbitol and mannitol or the like. Examples of buffers are citric acid, sodium hydrogenphosphate, glacial acetic acid and trometamol or the like. Examples of pH adjusters are hydrochloric acid, citric acid, phosphoric acid, acetic acid, sodium hydroxide, sodium carbonate and sodium hydrogencarbonate or the like. An example of soothing agents is benzyl alcohol or the like. Examples of preservatives are benzalkonium chloride, benzethonium chloride, p-hydroxybenzoate esters, sodium benzoate and chlorobutanol or the like.
The present invention also provides a method of treating ophthalmopathy containing administering the injection for ocular tissues containing the drug-PEG in an effective amount for treatment to the ocular tissues of patients.

Brief Description of Drawings

Fig. 1 is a graph showing changes in concentration (56 days) in a vitreous body.
Fig. 2 is a graph showing changes in concentration (56 days) in a retina.

Best Mode for Carrying out the Invention

Examples of the present invention are shown below, and they are intended for better understanding the present invention but are not to limit the scope of the present invention.

Synthesis Example

Synthesis of betamethasone-PEG conjugate

Methylene chloride (7 ml) was added to a mixture of methoxy-PEG-propionic acid [manufactured by Shearwater Polymers Co., Ltd., average molecular weight: ca. 5,000] (1.00 g; ca. 0.20 mmol) and dicyclohexylcarbodiimide (49.2 mg; 0.23 mmol) under a nitrogen atmosphere, and the whole was stirred at 0°C for 20 minutes. Next, betamethasone (59.3 mg; 0.15 mmol) and 4-dimethylaminopyridine (12.6 mg; 0.10 mmol) were added thereto, and the whole was stirred at room temperature overnight. The reaction mixture was put into a 30-fold amount of diethyl ether, and the resulting precipitate was filtered off and washed with small amounts of cold acetone and diethyl ether to give 1.02 mg of the target compound represented by the following formula as white crystals. Its yield was about 70%.

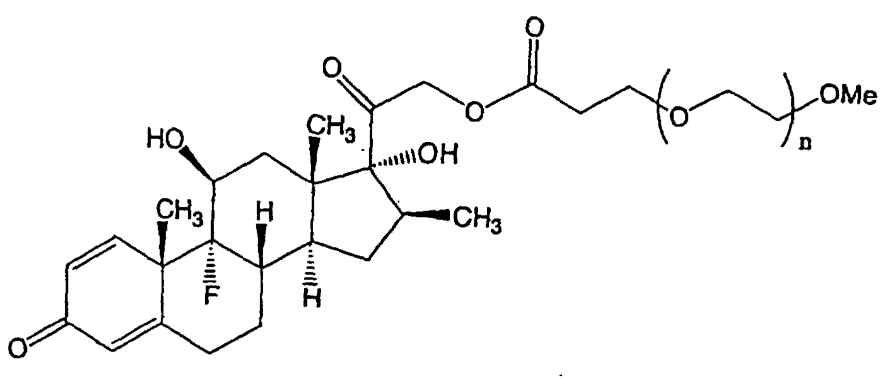

Intraocular pharmacokinetics tests

Intraocular pharmacokinetic tests by fluorophotometry in rabbits

It is difficult in terms of measurement techniques to actually trace transfer of slight amounts of tissues using the drug-PEG conjugate prepared above. Accordingly, fluorescein, which has fluorescence and can be measured with high sensitivity, was used as a model drug, a fluorescein-PEG conjugate (hereinafter referred to as "FL-PEG") was synthesized, and intraocular pharmacokinetics tests were performed. Fluorescein sodium (hereinafter referred to as "FL") was used as a comparative substance.

Synthesis of FL-PEG:

$NH_2$-PEG-propionic acid [manufactured by Shearwater Polymers Co., Ltd.] (1.00 g; ca. 0.20 mmol) and triethylamine (55.6 µl; 0.40 mmol) were dissolved in methanol (100 ml) under a nitrogen atmosphere. PEG having average molecular weight of about 5,000 was used. Next, fluorescein isothiocyanate (233 mg; 0.60 mmol) was added to the solution, and the whole was stirred at room temperature overnight. The reaction mixture was concentrated under reduced pressure to dryness, then the resulting solid matter was dissolved in chloroform/methanol (1/1, v/v), and unreacted fluorescein isothiocyanate was removed by column chromatography. The fractionated fraction was concentrated, the concentrate was put into a 30-fold amount of diethyl ether, and the resulting precipitate was filtered off and washed with a small amount of diethyl ether to give 0.86 g of the target compound represented by the following formula as pale yellow crystals.

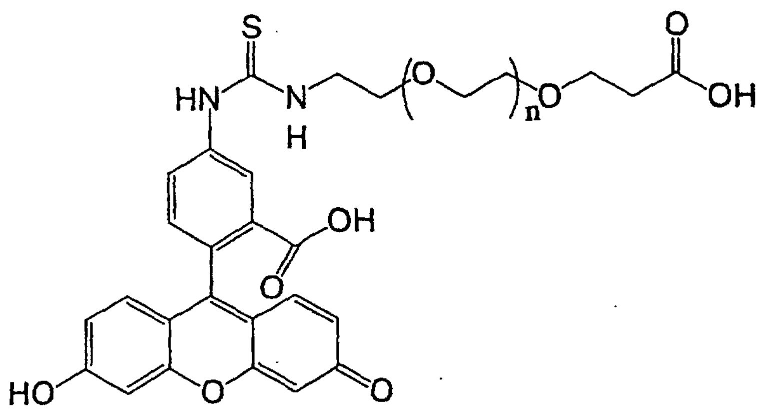

Preparation of liquid preparations:

A 2.6% sterilized glycerin solution (10 ml) was added to FL-PEG (18 mg), and this liquid was stirred to dissolve FL-PEG to prepare an injection. A 10 µg/ml injection containing FL was prepared in the similar manner.

Method of administration and method of measurement:

1) A mixed solution containing an aqueous ketamine hydrochloride solution (50 mg/ml) and an aqueous xylazine hydrochloride (50 mg/ml) in a ratio of 7:3 was administered intramuscularly to white rabbits to anesthetize.
2) A tropicamide (0.5%)/phenylephrine hydrochloride (0.5%) ophthalmic solution was instilled into eyes to cause mydriasis in the eyes.
3) The eyes were anesthetized with an oxybuprocaine hydrochloride (0.5%) ophthalmic solution.
4) The above-mentioned FL-PEG or FL liquid preparation was injected into the center of a vitreous body of one eye from an ocular pars plana in an amount of 100 µl respectively with a syringe equipped with a 30G needle.
5) Intraocular fluorescence intensity was measured with a fluorophotometry apparatus with the laps of time immediately after the administration to the vitreous body and at 1, 2, 4, 7, 15, 18, 23, 28, 35, 42, 49 and 56 days. A calibration curves were prepared, changes in concentration in the vitreous body and a retina were determined. Next, respective half-lives were calculated from the changes in concentration by a moment method. The above-mentioned operation 2) was carried out before the intraocular fluorescence intensity was measured.

Results:

Fig. 1 is a graph showing the changes in concentration in the vitreous body. Fig. 1 shows that FL was not detected on second day after the administration or later, whereas FL-PEG was detected even on 56th day and existed in the vitreous body. Fig. 2 is a graph showing the changes in concentration in the retina. Fig. 2 shows that FL was detected only on first day after the administration, whereas FL-PEG is detected even on 56th day and existed in the retina.
Next, Table 1 shows half-lives of FL and FL-PEG in the vitreous body and the retina. The half-life of FL-PEG

in the vitreous body is 3.4 days, whereas that of FL is only less than four hours. It means that FL-PEG prolonged the retention period in the vitreous body remarkably. The half-life of FL-PEG in the retina was 11.0 days. Since FL was detected only one point in time immediately after the administration to the retina, its half-life could not be calculated. However, it is clear that the half-life is quite short. Accordingly, it is guessed that FL-PEG migrates from the vitreous body to the retina and is retained there for a long period.

Table 1

| (Half-lives in vitreous body and retina) | | |
|---|---|---|
| Test substance | Vitreous body | Retina |
| FL-PEG | 3.4 days | 11.0 days |
| FL | <4 hours | - |

The numerical values in the table are the average of three samples respectively. The half-life of FL-PEG in the vitreous body was calculated using measured values on forth to 28th day. The half-life of FL-PEG in the retina was calculated using measured values on first to 56th day. The half-life of FL in the vitreous body was calculated using measured values immediately after the administration and on first day after the administration.

Pharmacological tests

1. Effects of intravitreously administered betamethasone-PEG conjugate on krypton laser-induced choroidal neovascularization in rats

The betamethasone-PEG conjugate (hereinafter referred to as "BM-PEG") prepared according to Example was administered intravitreously to rats, and effects on krypton laser-induced choroidal neovascularization were studied.

Preparation of liquid preparation:

Sterilized physiological saline (1 ml) was added to BM-PEG (100 mg), and this liquid is stirred to dissolve BM-PEG to prepare an injection for administration to vitreous bodies.

Method of administration and method of measurement:

1) A one ml/kg mixed solution containing an aqueous ketamine hydrochloride solution (50 mg/ml) and an aqueous xylazine hydrochloride solution (50 mg/ml) in a ratio of 7:1 was administered intramuscularly to rats to anesthetize them systemically.
2) A 0.5% tropicamide-0.5% phenylephrine hydrochloride solution was instilled into the eyes to cause mydriasis, and then photocoagulation was performed with a krypton laser photocoagulation apparatus (red) under coagulation conditions of spot size of 100 μm, output of 100 mW and coagulation time of 0.1 sec.
3) Cover glass for observing tissues was used as a contact lens, the photocoagulation was carried out in a posterior pole portion of ocular fundus at eight spots per eye sparsely avoiding thick retinal vessels. The photocoagulation was carried out focusing on the retinal posterior portion in order to cut a Bruch's membrane. After the photocoagulation, the ocular fundus was photographed.
4) The above-mentioned BM-PEG or a vehicle (physiological saline) was injected into the center of vitreous bodies of both eyes through an ocular pars plana in an amount af 5 μl respectively with a syringe equipped with a 33G needle.
5) On 14th day after the photocoagulation, 0.1 ml of 10% fluorescein was injected into tails intravenously, and fluorescein fundus angiography was performed.
6) In the fluorescein fundus angiography, a photocoagulation site where fluorescence diapedesis was not observed was judged as negative, and a photocoagulation site where apparent fluorescence diapedesis was observed was judged as positive. When there are two photocoagulation sites where a little fluorescence diapedesis was observed, they were judged as positive. Each neovascularization exhibition rate was calculated according to the following equation.

$$\text{Neovascularization exhibition rate (\%)} = \text{(Positive photocoagulation site number/Total photocoagulation site number)} \times 100$$

Results:

Table 2 shows an effect of intravitreously administered BM-PEG on choroidal neovascularization. While a neovascularization exhibition rate in vehicle administration eyes was 75.0%, the neovascularization exhibition rate in BM-PEG administration eyes was 35.4%. Thus the neovascularization exhibition was inhibited by the intravitreous administration of BM-PEG.

Table 2

| Test substance | Neovascularization exhibition rate (%) |
|---|---|
| Vehicle | 75.0 |
| BM-PEG | 35.4 |

The numerical values in the table are the average of seven eyes for the vehicle and six eyes for BM-PEG respectively.

2. Effects of subconjunctivally administered betamethasone-PEG conjugate on krypton laser-induced choroidal neovascularization in rats

The betamethasone-PEG conjugate (hereinafter referred to as "BM-PEG") prepared according to example was administered subconjunctivally to rats, and effects on krypton laser-induced choroidal neovascularization were studied. Betamethasone phosphate (hereinafter referred to as "BP") was used as a comparative substance.

Preparation of liquid preparations:

Sterilized physiological saline (1 ml) was added to BM-PEG (40 mg), and this liquid is stirred to dissolve BM-PEG to prepare an injection for subconjunctival administration. A 2.8 mg/ml injection containing BP was prepared in the similar manner.

Method of administration and method of measurement:

A method of administration and a method of measurement were carried out by changing the item 4) of the above-mentioned intravitreous administration tests as follows.

4) The above-mentioned BM-PEG or BP was injected subconjunctivally into both eyes in an amount of 50 µl respectively with a syringe equipped with a 30G needle.

Results:

Table 3 shows an effect of subconjunctivally administered BM-PEG on choroidal neovascularization. While a neovascularization exhibition rate in vehicle administration eyes was 71.9%, the neovascularization exhibition rate in BM-PEG administration eyes was 35.4%. Thus the neovascularization exhibition was inhibited by the subconjunctival administration of BM-PEG. A neovascularization exhibition rate of BP, which is not bonded to PEG, was 50.0%. Though an inhibitory effect of BP was observed, it was lower than that of BM-PEG. These results showed that the retention period in the ocular tissues was prolonged, and the inhibitory effect was increased by forming the PEG conjugate.

Table 3

| Test substance | Neovascularization exhibition rate (%) |
|---|---|
| Vehicle | 71.9 |
| BM-PEG | 35.9 |

Table 3 (continued)

| Test substance | Neovascularization exhibition rate (%) |
|---|---|
| BP | 50.0 |

The numerical values in the table are the average of eight eyes respectively.

Industrial Applicability

Drugs can be retained for a long period in ocular tissues such as an iris, a ciliary body, a vitreous body, a retina and an optic nerve by using injections for ocular tissues containing a drug-PEG conjugate of the present invention. Accordingly, the injections for ocular tissues of the present invention make it possible to treat or prevent diseases in various ocular tissues over a long period by a single administration.

## Claims

1. An injection for ocular tissues comprising a drug-polyethylene glycol conjugate.

2. The injection for ocular tissues as claimed in claim 1, wherein a method of injection into the ocular tissues is a subconjunctival injection, an intravitreous injection, a subretinal injection, an intrascleral injection, an anterior chamber injection or a sub-Tenon's injection.

3. The injection for ocular tissues as claimed in claim 1, wherein the drug is an anti-inflammatory, an immunosuppressor, an antiviral, an antimicrobial, an antimycotic, an antitumor, a nerve-protecting drug, a bloodflow-improving drug, an antiglaucomatous drug, an analgesic, an anesthetic, an angiogenesis inhibitor or a diagnostic agent.

4. The injection for ocular tissues as claimed in claim 1, wherein the drug is one for treatment or prevention of ophthalmopathy.

5. A method of treating ophthalmopathy containing administering an injection for ocular tissues containing a drug-polyethylene glycol conjugate to the ocular tissues of a patient in an effective amount for treatment.

6. The method of treating ophthalmopathy as claimed in claim 5, wherein a method of administration to the ocular tissues is a subconjunctival injection, an intravitreous injection, a subretinal injection, an intrascleral injection, an anterior chamber injection or a sub-Tenon's injection.

7. The method of treating ophthalmopathy as claimed in claim 5, wherein the drug is an anti-inflammatory, an immunosuppressor, an antiviral, an antimicrobial, an antimycotic, an antitumor, a nerve-protecting drug, a bloodflow-improving drug, an antiglaucomatous drug, an analgesic, an anesthetic, an angiogenesis inhibitor or a diagnostic agent.

8. The method of treating ophthalmopathy as claimed in claim 5, wherein the drug is one for treatment or prevention of ophthalmopathy.

9. Use of a drug-polyethylene glycol conjugate in the manufacture of an injection for ocular tissues.

10. The use as claimed in claim 9, wherein a method of injection into the ocular tissues is a subconjunctival injection, an intravitreous injection, a subretinal injection, an intrascleral injection, an anterior chamber injection or a sub-Tenon's injection.

11. The use as claimed in claim 9, wherein the drug is an anti-inflammatory, an immunosuppressor, an antiviral, an antimicrobial, an antimycotic, an antitumor, a nerve-protecting drug, a bloodflow-improving drug, an antiglaucomatous drug, an analgesic, an anesthetic, an angiogenesis inhibitor or a diagnostic agent.

12. The use as claimed in claim 9, wherein the drug is one for treatment or prevention of ophthalmopathy.

**Fig.1**

Change in concentration in vitreous body

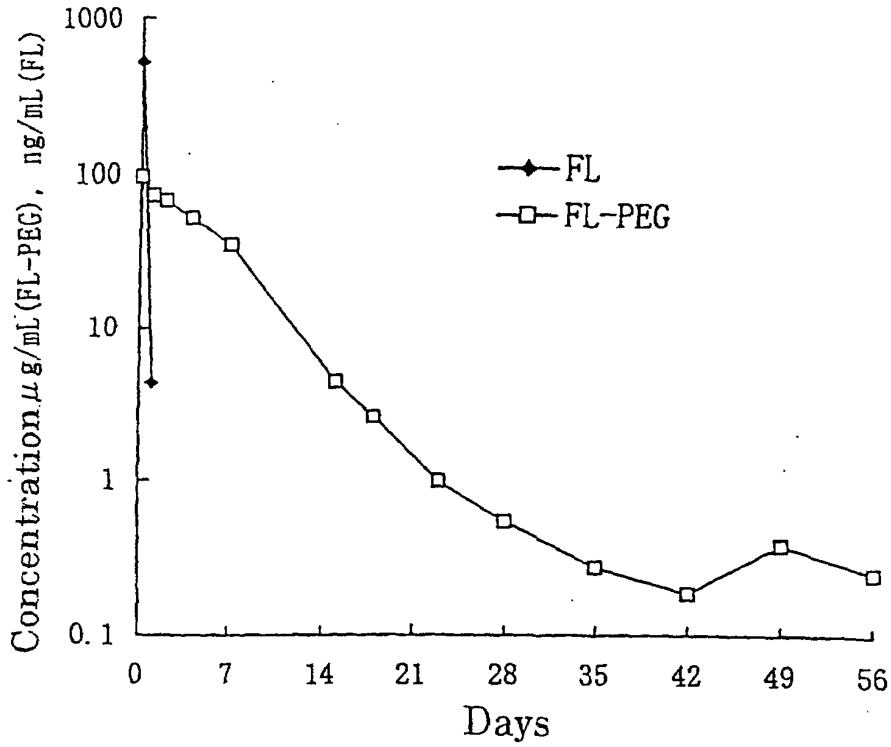


**Fig.2**

Change in concentration in retina

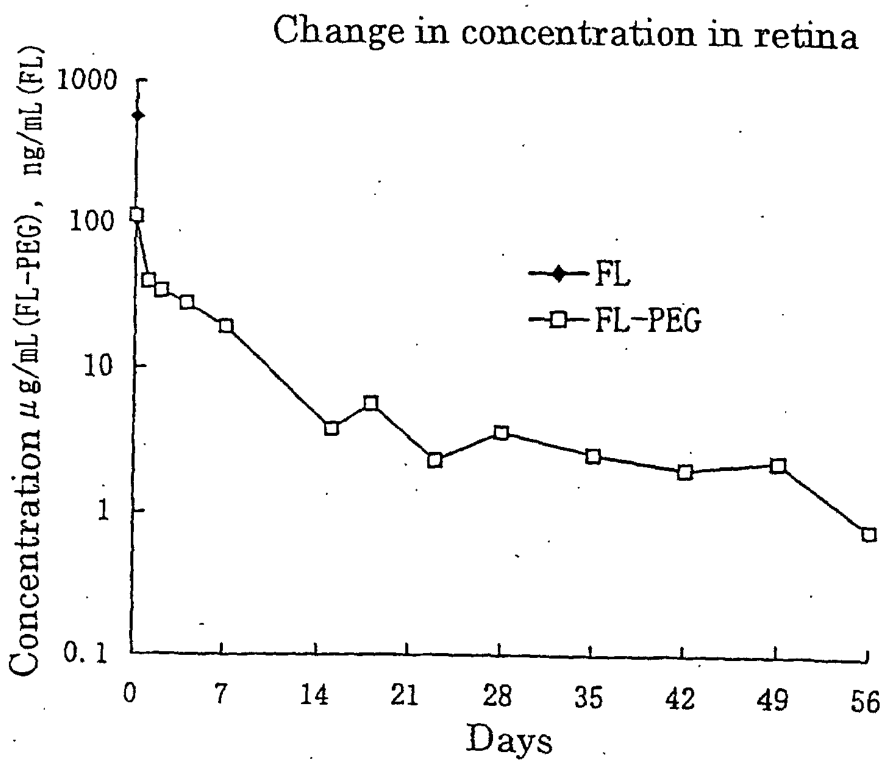

# INTERNATIONAL SEARCH REPORT

International application No.
PCT/JP02/10123

A. CLASSIFICATION OF SUBJECT MATTER
Int.Cl[7] A61K47/48, 47/34, 45/00, 9/08, A61P27/02, 27/06

According to International Patent Classification (IPC) or to both national classification and IPC

B. FIELDS SEARCHED

Minimum documentation searched (classification system followed by classification symbols)
Int.Cl[7] A61K47/48, 47/34, 45/00, 9/08, A61P27/02, 27/06

Documentation searched other than minimum documentation to the extent that such documents are included in the fields searched

Electronic data base consulted during the international search (name of data base and, where practicable, search terms used)
CAPLUS(STN), MEDLINE(STN), EMBASE(STN), BIOSIS(STN)

C. DOCUMENTS CONSIDERED TO BE RELEVANT

| Category* | Citation of document, with indication, where appropriate, of the relevant passages | Relevant to claim No. |
|---|---|---|
| X | WO 00/45835 A1 (Human Genome Sciences, Inc.), 10 August, 2000 (10.08.00), Particularly, abstract; Claims; page 57, line 8 to page 64, line 12; page 124, line 30 to page 125, line 5 & EP 1156820 A1 & JP 2002-539082 A | 1-4,9-12 |
| P,X | WO 01/74400 A1 (Santen Pharmaceutical Co., Ltd.), 11 October, 2001 (11.10.01), Particularly, abstract; Claims; page 12, line 17 to page 24, line 16 & JP 2002-326962 A | 1-4,9-12 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 January, 2003 (14.01.03) | 28 January, 2003 (28.01.03) |
| Name and mailing address of the ISA/ Japanese Patent Office | Authorized officer |
| Facsimile No. | Telephone No. |

Form PCT/ISA/210 (second sheet) (July 1998)

## INTERNATIONAL SEARCH REPORT

International application No.
PCT/JP02/10123

C (Continuation). DOCUMENTS CONSIDERED TO BE RELEVANT

| Category* | Citation of document, with indication, where appropriate, of the relevant passages | Relevant to claim No. |
|---|---|---|
| A | WO 93/00076 A1 (Minesota Mining and Manufacturing Co.), 07 January, 1993 (07.01.93), Particularly, page 6, line 28 to page 7, line 7; example 2(D) & EP 591284 A1 & DE 4120760 A1 & JP 6-508369 A | 1-4,9-12 |

Form PCT/ISA/210 (continuation of second sheet) (July 1998)

**INTERNATIONAL SEARCH REPORT**

International application No.
PCT/JP02/10123

**Box I Observations where certain claims were found unsearchable (Continuation of item 2 of first sheet)**

This international search report has not been established in respect of certain claims under Article 17(2)(a) for the following reasons:

1. [X] Claims Nos.: 5 to 8
because they relate to subject matter not required to be searched by this Authority, namely:
Claims 5 to 8 pertain to a method for treatment of the human body by therapy and thus relate to a subject matter which this International Searching Authority is not required, under the provisions of Article 17(2)(a)(i) of (continued to extra sheet)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)

**INTERNATIONAL SEARCH REPORT**

International application No.
PCT/JP02/10123

Continuation of Box No.I-1 of continuation of first sheet(1)

the PCT and Rule 39.1(iv) of the Regulations under the PCT, to make an international search.

Form PCT/ISA/210 (extra sheet) (July 1998)